# EUROPEAN PATENT APPLICATION

(11) **EP 1 849 878 A1**
(43) Date of publication of application: **31.10.2007**
(21) Application number: 06112978.9
(22) Date of filing: 24.04.2006
(51) Int. Cl.: C12Q 1/68, A61K 38/16

(54) **Means and methods for diagnosing and treating cancer based on the frmd3 gene**

(71) Applicant: DKFZ Deutsches Krebsforschungszentrum, 69120 Heidelberg (DE)
(72) Inventor: Angel, Peter, 69221 Dossenheim (DE); Hartenstein, Bettina, 69254 Malsch (DE); Haase, Doreen, 69115 Heidelberg (DE); Meister, Michael, 68309 Mannheim (DE); Muley, Thomas, 69509 Mörlenbach (DE)
(74) Representative: Dick, Alexander

(57) **Abstract**

The present invention relates to pharmaceutical compositions comprising a FRMD3 polynucleotide or polypeptide as well as diagnostic compositions, devices or kits comprising a FRMD3 polynucleotide or polypeptide as well as antibodies or oligonucleotides derived therefrom. Moreover, the present invention relates to methods and uses for diagnosing or treating a hyperproliferative disorder based on the aforementioned FRMD3 polynucleotides or polypeptides.

## Description

The present invention relates to pharmaceutical compositions comprising a FRMD3 polynucleotide or polypeptide as well as diagnostic compositions, devices or kits comprising a FRMD3 polynucleotide or polypeptide as well as antibodies or oligonucleotides derived therefrom. Moreover, the present invention relates to methods and uses for diagnosing or treating a hyperproliferative disorder based on the aforementioned FRMD3 polynucleotides or polypeptides.

Hyperproliferative disorders have in many cases a severe impact on the human or animal physiology. Many severe diseases, such as cancer, are caused by undesired, enhanced proliferation of cells. Specifically, the cancer diseases comprise some of the most life threatening medical conditions, such as lung carcinomas which are the leading cause of human cancer death.

Lung carcinomas, one of the most common cancers worldwide, are mainly caused by smoking tobacco and have a mortality of 75% (www.cancer.org). Human pulmonary cancers are divided into two major classes: small cell lung carcinoma (SCLC) and non small cell lung carcinoma (NSCLC). The latter accounting for about 80% of lung cancer cases (Greenlee 2000, CA Cancer J Clin 50: 7-33) consists of the three distinct histopathological subclasses pulmonary squamous cell carcinoma (pSCC), which exists in a cornified and non-cornified form, adenocarcinoma (pAC) and large cell carcinoma. Pulmonary cancers are usually diagnosed at late stages when, in addition to surgery, chemotherapy is a therapeutic necessity. This is especially problematic since patients then have an average age of 70 years (WHO) and suffer from multiple diseases, which makes treatment with cytotoxic drugs that have severe side effects impossible (Gridelli 2004, Cancer 101: 1733-1744).

Another life threatening cancer is colon carcinoma. Cancer of the colon and the rectum (colorectal cancer) is one of the major causes of cancer death worldwide and in Western society is second only to lung cancer. Only a small proportion (between 5 and 10%) of colorectal cancer cases are attributable to familial cancer syndromes and the majority seem to arise sporadically (Boyle and Lagman 2000, Br Med J 321: 805-808). Cancers of the colon accounted for about 1 million new cases in 2002 (9.4% of the world total). Numbers were not so different in men and women (ratio, 1.2:1). In terms of incidence, colorectal cancers rank fourth in frequency in men and third in women. Survival estimates (in men) at 5 years are 65% in North America, 54% in Western Europe, 34% in Eastern Europe, and 30% in India. The overall relatively good prognosis means that mortality is about one half that of incidence (about 529,000 deaths in 2002), while prevalence is second only to that of breast cancer worldwide, with an estimated 2.8 million persons alive with colorectal cancer diagnosed within 5 years of diagnosis (Parkin 2005, CA Cancer J Clin 55: 74 -108).

One class of biomarkers with functional relevance for various hyperproliferative disorders and specifically for cancer diseases are the so called tumor suppressor genes. The products of said tumor suppressor genes are required under physiological conditions to control the proliferation of cells expressing theses genes. A possible cause of a hyperproliferative disorder such as cancer, thus, may be the loss of the physiological function of the tumor supressor gene product. Putative tumor supressor genes are found among various protein families including the so called 4.1 superfamily. This superfamily is characterized in that the members share a highly conserved FERM domain, a 30kDa N-terminal membrane binding domain which suggests that the proteins are involved in membrane cytoskeletal interactions. The 4.1 superfamily comprises more than 40 members which have been subdivided into 5 subgroups (Sun 2002, J Cell Sci 115:3991-4000). However, the available information as to whether such tumor supressor genes can be used for therapeutic approaches is rather limited and restricted.

Thus, although highly desired, reliable and efficient therapeutic and diagnostic approaches are not yet available.

Therefore, the technical problem underlying the present invention may be seen in the provision of means and methods for treating or diagnosing hyperproliferative disorders reliably and efficiently. Moreover, the drawbacks according to the prior art shall be avoided.
The technical problem is solved by the embodiments characterized in the claims and herein below.

Accordingly, the present invention relates to a pharmaceutical composition comprising
(i) a polynucleotide comprising
   a) a nucleic acid having a sequence as shown in SEQ ID NO: 1,
   b) a nucleic acid encoding a polynucleotide as shown in SEQ ID NO: 2,
   c) a nucleic acid which is at least 60% identical to the nucleic acid of a) or b),
   d) a nucleic acid which encodes a protein being at least 60% identical to the protein encoded by any one of a) to c), and
   e) a nucleic acid encoding a biologically active fragment of any one of a) to d) or
(ii) a polypeptide encoded by the polynucleotides of (i) and, optionally, a pharmaceutically acceptable carrier.

The term "pharmaceutical composition" as used herein refers to the polynucleotides or polypeptides of the present invention and optionally one or more pharmaceutically acceptable carrier(s) or additional drugs which may or may not act either synergistically or additively.

The polynucleotides or polypeptides of the present invention may be formulated as pharmaceutically acceptable salts. Acceptable salts comprise acetate, methylester, HC1, sulfate, chloride and the like. If a gene therapy approach is envisaged, the polynucleotides of the present invention may be formulated, e.g., as retroviruses, as retroviral vectors or as liposomes. The pharmaceutical compositions can be adopted to the mode of administration comprising the forms of tablets, capsules, suppositories, solutions, suspensions, liposomes, vectors, viruses or the like.

The pharmaceutical compositions can be conveniently administered by any suitable route conventionally used for drug administration, for instance, orally, topically, parenterally or by inhalation. Whether a route for administration is suitable depends on the nature of the pharmaceutical composition, i.e., pharmaceutical compositions comprising a polynucleotide of the invention may, preferably, be administered by routes suitable for gene therapeutic carriers.

The polynucleotides or polypeptides may be administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable character or diluent is dictated by the amount of active ingredient with which it is to be combined, the route of administration and other well-known variables. The carrier(s) must be "acceptable" in the sense of being compatible with the other ingredients of the formulation and not deleterious to the recipient thereof. The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. The diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like. It is to be understood that the pharmaceutical compositions shall comprise the polynucleotides or polypeptides of the present invention in a therapeutically effective dose. A therapeutically effective dose refers to that amount of the substance according to the invention which ameliorate the symptoms or condition. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, gender, time and route of administration, general health, and other drugs being administered concurrently. Progress can be monitored by periodic assessment. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. However, depending on the subject and the mode of administration, the quantity of substance administration may vary over a wide range to provide from about 0.01 mg per kg body mass to about 10 mg per kg body mass.

The pharmaceutical compositions and formulations referred to herein are administered at least once in accordance with the use of the present invention. However, the said pharmaceutical compositions and formulations may be administered more than one time, for example from one to four times daily up to a non-limited number of days.

The polynucleotides or polypeptides can be administered in combination with other substances either in a common pharmaceutical composition or as separated pharmaceutical compositions.

The term "polynucleotide" as used herein relates to nucleic acid molecules and, thus, encompasses genomic DNA, cDNA, or RNA.

The nucleic acid molecules shall comprise the specific nucleotide sequence shown in SEQ ID NO: 1 (i.e. the human FRMD3 cDNA). It is to be understood that the nucleic acid molecules may comprise additional nucleotides. Such nucleic acid molecules represent either the FRMD3 gene comprising introns, exons as well as untranslated regions or expression control sequences. However, the nucleic acid molecule may also, preferably, be comprised by a vector, plasmid or targeting construct. Moreover, the polynucleotide of the present invention encompasses variants of the aforementioned nucleic acid molecules comprising the specific nucleic acid sequence. Such variants may be due to the degenerated genetic code, i.e. comprise a nucleic acid encoding an amino acid sequence as shown in SEQ ID NO: 2 (i.e., the human FRMD3 polypeptide). Further, variants may be orthologs or paralogs from a different species or homologs. It is envisaged that the aforementioned variants comprise a nucleic acid sequence being at least 60%, at least 70%, at least 80%, at least 90%, at least 95%, at least 96%, at least 97%, at least 98% or at least 99% identical to the specific nucleotide sequence of SEQ ID NO: 1. Furthermore, variants as meant herein are those polynucleotides which comprise a nucleic acid sequence encoding an amino acid sequence whichh is at least 60%, at least 70%, at least 80%, at least 90%, at least 95%, at least 96%, at least 97%, at least 98% or at least 99% identical to the specific amino acid sequence of SEQ ID NO: 2. Identiy of nucleic acid sequences or amino acid sequences can be determined by various algorithms available in the prior art. Preferably, sequence identity as referred to in accordance with the present invention is determined by, preferably, using GAP (Needleman and Wunsch, J. Mol. Biol. 48; 443-453(1970)), BESTFIT (using the local homology algorithm of Smith and Waterman (Advances in Applied Mathematics 2; 482-489 (1981))), BLAST (Atschul, S. F. , Gish, W., Miller, W. , Myers, E. W. & Lipman, D. J. , J. Mol. Biol. 215: 403-410 (1990)), FASTA or TFASTA (W. R. Pearson and D. J. Lipman Proc. Natl. Acad. Sci. USA 85: 2444- 2448 (1988)). It Is to be understood that, preferably, the default parameters for the algorithms are to be used for sequence comparison. The software for performing BLAST analysis is publicly available through the National Centre for Biotechnology Information. The abovementioned variants are, more preferably, identified using blast default parameters (BLOSUM62 matrix, gap opening penalty 11 and gap extension penalty 1) and, preferably, the full length sequences are used for analysis. Preferably, the aforementioned variants encode a polypeptide having essentially the same biological functions or immunological properties as the FRMD3 protein according to SEQ ID NO: 2. A polypeptide shall be deemed to have the same biological functions if it is capable of inducing apoptosis in a cell. Suitable assays for determining apoptosis are described in the accompanying Examples. A polypeptide shall have essentially the immunological properties of the FRMD3 polypeptide (preferably according to SEQ ID NO: 2) if the variant polypeptide comprises one or more epitopes which can be specifically recognized by a specific FRMD3 antibody. Polynucleotides encompass further those nucleic acids which encode biologically active fragments of the aforementioned polypeptides. Again, it is, preferably, envisaged that the said fragments shall exhibit at least essentially the immunological properties of the FRMD3 polypeptide as referred to above.

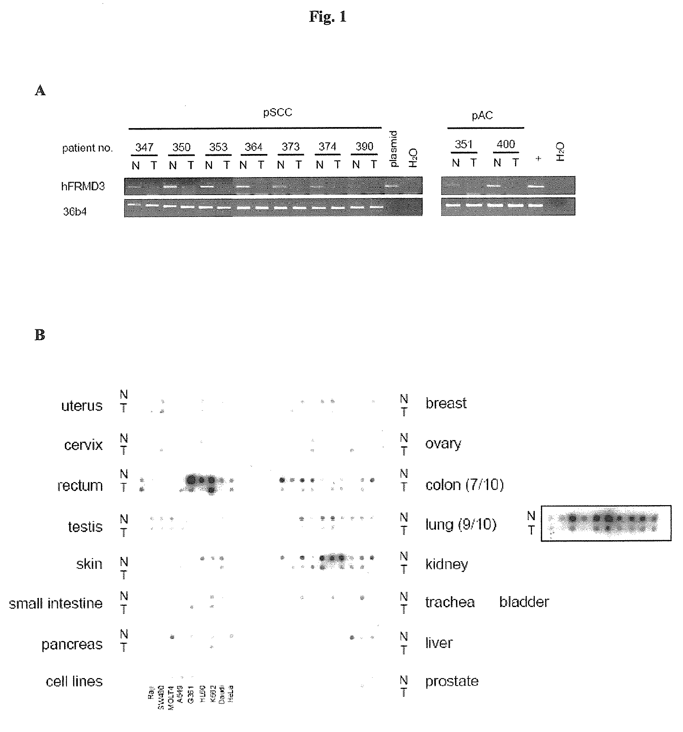

In general, FRMD3 (FERM domain containing 3) polynucleotides and the encoded polypeptides were first described by Ni 2003, J Human Genet 48: 101-106, who named it 4.10 since they found it specifically expressed in ovary tissue. Due to the homology of its N-terminal sequence to the FERM domain of known 4.1 proteins, it was classified as a 4.1 proteins. It is located on chromosome 9q21-9q22, consists of 14 exons with an open reading frame of 1,662 bp being translated into a 553 amino acid protein. Apart from ovary, cDNAs of FRMD3 have been found in the adult medulla of human brain (AAH37253) and in the hippocampus (AAH41376) as well in the placenta and choriocarcinoma (AAH23560) (NCBI nucleotide database). The fetal expression pattern includes skeletal muscle, thymus and brain. Yet, the function of FRMD3 remained completely unknown. FRMD3 belongs to the large superfamily of 4.1 protein, which comprises the band 4.1 proteins, the ERM proteins ezrin (a component of the microvilli cytoskeleton), radixin (an actin-modulating protein) and moesin, as well as talin-related molecules, protein tyrosine phosphatases (PTPH) and novel band 4.1-like 4 (NBL4) (Fig. 1A). The common feature of all these different molecules is the conserved FERM domain (Fig. 1B) although the binding specificity of this domain differs in the subfamilies (Chishti 1998, Trends Biochem Sci 23: 281-282). The name FERM is derived from the family members 4.1, ezrin, radixin and moesin. ERM, 4.1 and talin-related proteins also share their ability to bind to cytoskeletal proteins like actin. Structural analysis of the FERM domain of moesin revealed three subdomains F1, F2 and F3 (Edwards 2001, Biochemistry 40:7061-7068) that form a three-dimensional structure differing between family members (Sun 2002, loc. cit.). Interestingly, F1 shares homologies with ubiquitin, F2 with acyl-CoA-binding proteins and F3 with phosphotyrosine binding, pleckstrin homology (PH) and Enabled/VASP homology 1 domains. For ERM proteins, the mechanism responsible for regulation of their protein activities has also been unraveled. It depends on intermolecular and intramolecular associations that both involve the FERM domain, which can interact with itself or the FERM domain of other molecules. In addition, the N-terminal FERM domain can also bind the C-terminus of the same or other ERM molecules. When ERM proteins are phosphorylated by kinases like Rho kinase, the interaction of the N- and C-terminus is interrupted and the protein adopts an "open" form, which uncovers binding sites and is considered active. In contrast to this mechanism, merlin is only active in the "closed" form, in which the binding site is formed. The founding member of the band 4.1 protein family, 4.1R, was first identified in red blood cells where it is involved in maintaining the typical erythrocyt cell shape. The name 4.1 was taken from the position at which the protein was detected after 2D SDS PAGE (Holzwarth 1976, J Supramol Struct 4:161-168). The other band 4.1 family members described so far are 4.1G (generally expressed), 4.1B (brain) and 4.1N (neurons). All band 4.1 proteins are characterized by three highly conserved domains: a 30 kDa N-terminal FERM domain, an 8-10 kDa spectrin-actin-binding domain (SABD) and a C-terminal domain (CTD) of 22-24 kDa. In addition the proteins possess three unique domains (U1, U2 and U3) (Fig. 1C). The FERM domain of 4.1 proteins binds a variety of different partners including CD44, p55 calmodulin, glycophorin C and glycophorin D and Band 3 protein. The SABD domain was shown to not only interact with spectrin and actin but also tubulin. Furthermore binding of the CTD was shown to the immunophilin FKBP13 and the nuclear mitotic apparatus (NuMA) protein. The main functions of the 4.1 proteins require the interaction of these domains with the cytoskeleton and the cell membrane. By the binding of the FERM domain to the cytoplasmatic tail of membrane proteins and the simultaneous interactions of the SABD, membrane proteins are linked to the cytoskeleton and localized to certain areas of the cell or membrane. This stabilizes the cell membrane and thereby sustains the cell shape. All 4.1 proteins localize to the plasma membrane and are often found at regions of cell-cell contact. For 4.1B it has been shown that the U2 domain (Fig. 1C) is directly involved in the membrane localization of the protein.

In addition to the above definitions, a FRMD3 polynucleotide of the present invention may be the endogenous FRMD3 gene comprising a sequence as recited herein, wherein said FRMD3 gene is operatively linked to an exogenous expression control sequence, i.e. an expression control sequence which has been linked to the endogenous FRMD3 gene locus by DNA recombination techniques such as homologous recombination. In such a case, the pharmaceutical composition which is administered to a subject in need thereof will be a gene expression control sequence in a suitable targeting vector for homologous recombination. Such a targeting vector shall comprise fragments of the aforementioned specific FRMD3 polynucleotides which allow for homologous integration into the endogenous FRMD3 locus upstream of the transcription initiation site. Preferably, the targeting comprises a constitutive or inducible promoter, optionally with Enhancer/Silencer elements.

Moreover, the polynucleotide according to the present invention may be comprised by a vector. Suitable vectors are, preferably, phage, plasmid, viral or retroviral vector. Retroviral vectors may be replication competent or replication defective. In the latter case, viral propagation generally will occur only in complementing host cells. The polynucleotides may be joined to a vector containing selectable markers for propagation in a host. Generally, a plasmid vector is introduced in a precipitate such as a calcium phosphate precipitate or rubidium chloride precipitate, or in a complex with a charged lipid or in carbon-based clusters, such as fullerens. Should the vector be a virus, it may be packaged in vitro using an appropriate packaging cell line prior to application to host cells. More preferably, the vector comprises the polynucleotide operatively linked to an expression control sequence allowing expression in eukaryotic cells. Expression of said polynucleotide comprises transcription of the polynucleotide, preferably into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers. Possible regulatory elements permitting expression in eukaryotic host cells are the CMV-, SV40- , RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pCDM8, pRc/CMV, pcDNA1, pcDNA3 (In-vitrogene), pSPORT1 (GIBCO BRL). Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994). Alternatively, the polynucleotides and vectors of the invention can be reconstituted into liposomes for delivery to target cells.

The term "polypeptide" as used herein refers to a polypeptide having an amino acid sequence which is encoded by the FRMD3 polynucleotides referred to above. It is to be understood that the term also encompasses naturally occurring or chemically modified derivatives of said polypeptides. Naturally occurring derivatives can be obtained by, e.g., phosphorylation, glycosylation or myristylation. Chemically modified polypeptides can be obtained by all types of chemical modifications including those affecting the amino acid backbone of the polypeptide or the individual amino acid residues.

Advantageously, it has been found in the studies underlying the present invention that the biological activity of the FRMD3 polypeptide can be used to efficiently treat hyperproliferative disorders. Specifically, functional analysis was performed in HEK293 cells, which showed very low endogenous FRMD3 expression. Colony formation assays in cells transfected with FRMD3 fused to a myc-tag revealed a reduction in colony number to 25% of the mock control. This effect was not caused by differences in plating efficiency or a cell cycle arrest. Instead, FRMD3 expression lead to an increased rate of specific apoptosis in HEK293. Thus, FRMD3 induces apoptosis in such hyperproliferative cells and, specifically, in lung or colon cancer cells. Thereby, the undesired hyperproliferative cells can be effectively destroyed. In case of cancer cells, FRMD3 operates as a tumor suppressor gene.

The definitions and explanations of the terms made above and herein below apply for all embodiments described in this specification accordingly.

Moreover, the present invention relates to the use of a polynucleotide or polypeptide as defined above for the manufacture of a pharmaceutical composition for treating, preventing and/or ameliorating a hyperproliferative disorder.

The term "manufacture" as used herein encompasses, preferably, the preparation of the polynucleotide or polypeptide specified herein above in pharmaceutical acceptable form. It is to be understood that a polynucleotide or polypeptide may be formulated by various galvenic techniques including those explicitly mentioned above.

The term "hyperproliferative disorder" as used herein relates to any disease or disorder which is caused by an impaired proliferation of cells in a subject. Impaired proliferation as used herein means an increased proliferation which results in the disease or disorder condition, i.e. a pathological condition. Accordingly, cells which causes a hyperproliferative disorder as meant herein are characterized by an impaired proliferation control mechanism. Moreover, the cells causing the said hyperoliferative disorder may also exhibit further biological characeristics such as an increased migration potential or an altered (pathological) cellular physiology or an altered metabolism. Whether a cell may cause a hyperproliferative disorder can be determined by the presence of suitable biomarkers. Suitable biomarkers for various hyperproliferative disorders are well known in the art.

Preferably, the hyperproliferative disorder referred to in accordance with the present invention is cancer. The term cancer includes benignant as well as malignat forms of cancer. More preferably, the cancer is a lung cancer or colorectal cancers referred to herein as colon cancer. A biomarker which can be used to determine cancer as used herein is FRMD3. As discussed in detail below, it has been found that the absence of FRMD3 transcripts or protein is a useful indicator for the development of cancer such as lung or colon cancer.

Preferably, a hyperproliferative disorder also encompasses neoplasia.

It is to be understood that compounds which act as agonists of the FRMD3 polynucleotides or polypeptides shall also be suitable as pharmaceutical compositions in general and, in particular, for treating, preventing and/or ameliorating a hyperoliferative disorder referred to herein above. Such FRMD3 agonists may be coumpounds which, in the case of polynucleotide agonists, may either increase the rate of transcription of the FRMD3 gene or which may stabilize the FRMD3 transcripts present in a cell. In case of FRMD3 polypeptides, agonists may be compounds which enhance the activity of the FRMD3 polypeptides or which prevent the FRMD3 polypeptides from degradation. Suitable compounds can be idenified out of polypeptide or peptide libraries, antibody libraries or small molecule libraries by various techniques. An agonist of the polynucleotide or polypeptide can be identified by the following steps: (a) contacting the compound suspected to be an agonist with a cell expressing the FRMD3 polynucleotide or containing the FRMD3 polypeptide; and (b) determining the amount of the FRMD3 polynucleotide or polypepide in said cell in comparison to a reference cell which has not brought into contact to the said compound. Alternatively, an increase in FRMD3 induced apoptosis upon contacting a cell population with a compound suspected to be an agonist shall be indicative for an agonistically acting compound. A suitable method for identification, thus, comprises the steps of: (a) contacting a cell population expressing FRMD3 with a compound suspected to act as an agonist of FRMD3; and (b) determining the apoptosis rate in said cell population in comparison to a reference which has not been brought into contact with the said compound whereby an increase in the apoptosis rate is indicative for an agonistically acting compound.

The present invention also relates to a diagnostic composition comprising the polynucleotide or polypetide as defined above, an antibody which specifically binds to the polypeptide defined above or an oligonucleotide specifically recognizing the polynucleotide as defined above.

The term "diagnostic composition" refers to one of the aforementioned compounds which is prepared to be used for diagnostic purposes, in principle. It is to be understood that depending on the nature of the diagnostic agent, i.e. depending on whether a polynucleotide, a polypeptide, an antibody or an oligonucleotide is used, the diagnostic composition may comprise additional agents such as agents which allow hybridization, antibody binding or detection. Such additional agents are well known to those skilled in the art.

The term "antibody" refers to polyclonal and monoclonal antibodies, as well as fragments thereof, such as Fv, Fab and F(ab)₂ fragments that are capable of binding antigen or hapten. The present invention also includes humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art.

The term "oligonucletide" refers to a nucleic acid which is derived from the polynucleotides referred to above or the reverse complementary sequence thereof. The oligonucleotide shall be of sufficient length in order to be capable of hybridizing to the aforementioned polynucleotides under stringent hybridization conditions. Whether hybridization conditions are stringent can be determined by methods well known in the art (Hames and Higgins 1985, Nucleic Acid Hybridization: A Practical Approach. IRL Press, Oxford/ USA; McPherson, Quirke and Taylor 1991, PCR 1-3: A Practical Approach. IRL Press, Oxford/ USA). Stringent hybridization conditions are, preferably, those conditions used for RT-PCR as described in the accompanying Examples. Preferably, an oligonucleotide comprises between 5 and 100 nucleotides in length of the sequences of the aforementioned polynucleotides. More preferably, an oligonucleotide consists essentially of 10 to 40, 10 to 30, 10 to 25, 12 to 25, 15 to 25, 18 to 25 or 20 to 25 nucleotides in length of the aforementioned polynucleotides. Most preferably, the oligonucleotide has a nucleic acid sequence as set forth in SEQ ID NO: 3 or 4.

It has been found in accordance with the studies underlying the present invention that the amount of FRMD3 transcripts or polypeptide in a sample of a subject is indicative for a hyperproliferative disorder or an increased risk of suffering therefrom in the future. Specifically, the FRMD3 gene was found to be downregulated in 20 out of 23 NSCLC. Consequently, the absence or reduced expression of the FRMD3 gene is a reliable biomarker for hyperproliferating cells and, thus, for the disorders referred to in accordance with the present invention. Thus, thanks to the present invention, a hyperproliferative disorder such as cancer can be more reliably and efficiently detected. Moreover, the diagnostic means provided in accordance with the present invention which are described in this specification are suitable for large scale screenings as well as for an individual or even a near-patient diagnosis.

Further, the present invention encompasses a diagnostic device for diagnosing a hyperproliferative disorder comprising the polynucleotide or polypeptide as defined above, an antibody which specifically recognizes the polypeptide as defined above or an oligonucleotide specifically recognizing the polynucleotide as defined above.

The term "device" as used herein relates to a system of means comprising at least the aforementioned means operatively linked to each other as to allow the diagnosis. Preferred means for determining the amount of the FRMD3 polynucleotide or polypeptide are the polynucleotide, polypeptide, antibody which specifically recognizes the polypeptide or an oligonucleotide specifically recognizing the polynucleotide as describe above. How to link the means in an operating manner will depend on the type of means included into the device. For example, where means for automatically determining the amount of the FRMD3 polynucleotide or polypeptide are applied, the data obtained by said automatically operating means can be processed by, e.g., a computer program in order to diagnose an hyperprolifertaive disorder referred to herein.

Preferably, the means are comprised by a single device in such a case. Said device may accordingly include an analyzing unit for the measurement of the amount of the polynucleotide or polypeptide in an applied sample (e.g., an microarray or biochip) and a computer unit for processing the resulting data for the prediction.

Alternatively, where means such as test stripes are used for determining the amount of, e.g., the FRMD3 polypeptide, the means for diagnosing may comprise control stripes or tables allocating the determined amount to an amount known to be accompanied with a hyperproliferative disorder recited herein or an amount known to be indicative for a healthy subject which does not suffer from the said hyperproliferative disorder. The test stripes are, preferably, coupled to a detecing agent which specifically binds, e.g., to the FRMD3 polypeptide as describe elswhere in this specification. In the case of test strips as diagnostic devices, it is to be understood that the means are operatively linked in that the user of the system brings together the result of the determination of the amount and the diagnostic value thereof due to the instructions and interpretations given in a manual. Preferred devices are those which can be applied without the particular knowledge of a specialized clinician, e.g., test stripes or electronic devices which merely require loading with a sample. Alternatively, the results may be given as output of diagnostic raw data which, however, may need interpretation by the clinician.

The diagnostic devices or compositions described herein above are, preferably, used for diagnosing a hyperproliferative disorder. Depending on the nature of the devices or compositions, they can be applied in various techniques. Nevertheless, it is to be understood that the present invention, in principle, also relates to the use of the polynucleotide or polypetide as defined above, an antibody which specifically recognizes the polypeptide defined above or an oligonucleotide which specifically recognizese the polynucleotide as defined above for the preparartion of a diagnostic composition for diagnosing a hyperproliferative disorder in accordance with the present invention and, preferably, for diagnosing lung or colon cancer.

Also, specifically contemplated by the present is a method for diagnosing a hyperproliferative disorder in a subject comprising the steps of:
a) determining the amount of the polynucleotide or polypeptide as defined above in a sample of a subject; and
b) comparing said amount to a reference amount whereby the hyperproliferative disorder is diagnosed.

The term "diagnosing" as used herein refers to assessing the probability according to which a subject is suffering or will suffer from a hyperproliferative disorder. Thus, the term encompasses the determination of a hyperproliferative disorder as well as the prediction of whether a subject is at risk for developing a hyperproliferative disorder. As will be understood by those skilled in the art, such an assessment is usually not intended to be correct for 100% of the subjects to be diagnosed. The term, however, requires that a statistically significant portion of subjects can be properly diagnosed. Whether a portion is statistically significant can be determined without further ado by the person skilled in the art using various well known statistic evaluation tools, e.g., determination of confidence intervals, p-value determination, Student's t-test, Mann-Whitney test etc.. Details are found in standard text books such as Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "subject" as used herein relates to animals, preferably mammals, and humans.

The term "amount" as used herein encompasses the absolute amount of the FRMD3 polynucleotide or polypeptide, the relative amount or concentration of the FRMD3 polynucleotide or polypeptide as well as any value or parameter which correlates thereto. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., expression levels determined from biological read out systems in response to the peptides or intensity signals obtained from specifically bound agents. It is to be understood that values correlating to the aforementioned amounts or parameters can also be obtained by all standard mathematical operations.

Determining the amount of a FRMD3 polynucleotide or polypeptide according to the present invention relates to measuring the amount or concentration, preferably semi-quantitatively or quantitatively. Measuring can be done directly or indirectly. Direct measuring relates to measuring the amount or concentration of the FRMD3 polynucleotide or polypeptide based on a signal which is obtained from the FRMD3 polynucleotide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the FRMD3 polynucleotide or polypeptide. Indirect measuring includes measuring of a signal obtained from a secondary component (i.e. a component not being the natriuretic peptide itself) or a biological read out system, e.g., measurable cellular responses, ligands, labels, or enzymatic reaction products.

In accordance with the present invention, determining the amount can be achieved by all known means for determining the amount of a polynucleotide or polypeptide in a sample. Said means comprise immunoassay devices and methods which may utilize labled molecules in various sandwich, competition, or other assay formats. Said assays will develop a signal which is indicative for the presence or absence of the FRMD3 polynucleotides or polypeptides. Moreover, the signal strength can, preferably, be correlated directly or indirectly (e.g. reverse- proportional) to the amount of polypeptide present in a sample. Further suitable methods comprise measuring a physical or chemical property specific for the FRMD3 polynucleotide or polypeptide such as its precise molecular mass or NMR spectrum. Said methods comprise, preferably, biosensors, optical devices coupled to immunoassays, biochips, analytical devices such as mass-spectrometers, NMR- analyzers, or chromatography devices and those diagnostic devices recited elsewher in this specification. Further, methods include micro-plate ELISA-based methods, fully-automated or robotic immunoassays.

In a preferred embodiment, the method for determining the amount of a FRMD3 polynucleotide or polypeptide comprises the step of measuring a specific intensity signal obtainable from the FRMD3 polynucleotide or polypeptide in the sample. As described above, such a signal may be the signal intensity observed at an m/z variable specific for FRMD3 polynucleotide or polypeptide observed in mass spectra or a NMR spectrum specific for the FRMD3 polynucleotide or polypeptide.

In another preferred embodiment, the method for determining the amount of a FRMD3 polynucleotide or polypeptide comprises the steps of (a) contacting the peptide with a detection agent, (b) (optionally) removing non-bound ligand, (c) measuring the amount of bound ligand. The bound detecting agent will generate an intensity signal. Binding according to the present invention includes both covalent and non-covalent binding. A detecting agent according to the present invention can be any compound, e.g., a peptide, polypeptide, antibody, nucleic acid, or small molecule, binding to the FRMD3 polynucleotide or polypeptide. Preferred detecting agents for a FRMD3 polypeptide include antibodies as described elsewhere in this specification or peptides or polypeptides such as receptors or binding partners of the FRMD3 polypeptide. Such detecting agents may be, preferably, used in all kinds of binding, two-hybrid or immunoassays. Preferred detecting agents in the case of a FRMD3 polynucleotide encompass complementary polynucleotides or sequence specific oligonucleotides including primers for PCR detection. This type of detecting agents can be used, preferably, in hybridization assays, such as Southern or Northern Blots, or for PCR based techniques.

It is to be understood that the detecting agent binds specifically to the FRMD3 polynucleotide or polypeptide. Specific binding according to the present invention means that the agent should not bind substantially to ("cross-react" with) another peptide, polypeptide, polynucleide or any other compound present in the sample to be analyzed. Non-specific binding may be tolerable, if it can still be distinguished and measured unequivocally, e.g. according to a size signal on a Southern, Northern or Western Blot, or by its relatively higher abundance in the sample. Binding of the detecting agent can be measured by any method known in the art. Preferably, said method is semi-quantitative or quantitative.

The term "sample" refers to a sample of a body fluid, to a sample of separated cells or to a sample from a tissue or an organ. Samples of body fluids can be obtained by well known techniques. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the FRMD3 polynucleotide or polypeptide. More preferably, the sample in accordance with the present invention is a tissue sample of lung tissue or colon tissue.

The term "reference amount" as used herein refers to an amount which allows assessing whether a subject will suffer from a hyperproliferative disorder by a comparison as referred to above. Accordingly, the reference may either be derived from a subject suffering from a hyperproliferative disorder or a healthy subject. The reference amount applicable for an individual subject may vary depending on various physiological parameters such as age, gender, or subpopulation. Thus, a suitable reference amount may be determined by the method of the present invention from a reference sample to be analyzed together, i.e. simultaneously or subsequently, with the test sample.

Comparing as used herein encompasses comparing the amount of the FRMD3 polynucleotide or polypeptide comprised by the sample to be analyzed with an amount of a suitable reference source specified above. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically predict whether a subject is at risk of developing cardiovascular complications when suffering already from a bone mineral disorder. It has been found in accordance with the present invention that a significantly reduced amount or the absence of FRMD3 polypeptide or FRMD3 transcripts in a cell are indicative for a hyperproliferative disorder or an increased risk of developing said disorder. Thus, the absence of or a significantly reduced amount of either FRMD3 polypeptide or FRMD3 transcripts (polynucleotides) are indicative for a hyperproliferative disorder in a subject.

In the following, specifically preferred embodiments of the diagnostic method of the present invention are described.

In a preferred embodiment of the method of the present invention said reference amount is obtained from a subject known to suffer from a hyperproliferative disorder.

More preferably, an amount essentially identical or less than the reference amount is indicative for the hyperproliferative disorder.

In a further preferred embodiment of the method the reference amount is obtained from a subject known not to suffer from a hyperproliferative disorder.

More preferably, an amount less than the reference amount is indicative for a hyperproliferative disorder.

In another preferred embodiment of the method of the present said hyperproliferative disorder is cancer and, most preferably, a lung cancer or colon cancer.

Finally, the present invention relates to a kit for carrying out the method of the present invention comprising the polynucleotide or polypetide as defined above, an antibody which specifically recognizes the polypeptide defined in above or an oligonucleotide which specifically recognizes the polynucleotide as defined above and instructions for carrying out the said method.

The term "kit" as used herein refers to a collection of the aforementioned means, preferably, provided in separate containers or within a single container. The kit, also preferably, comprises instructions for carrying out the method of the present invention.

All references cited in this specification are herewith incorporated by reference with respect to their entire disclosure content and the disclosure content specifically mentioned in this specification.

The figures show:
**Figure 1**: (A) shows the results of a semiquantitative RT-PCR analysis of human FRMD3 expression in normal tissue (N) and tumor tissue (T) obtained from patients suffering from a pulmonary epithelial carcinoma (pSCC) or adenocarcinoma (pAC). RT-PCR has been normalized with respect to the ribosomal protein P0 (m36b4). The cDNA clone #IRATp970B0361D from the RZPD and the cDNA of patient number 350 served as positive controls. (B) Expressionanalysis of human FRMD3 in normal tissue (N) and tumor tissue (T) of the same patient with respet to the tumor type and expressionanalysis of various cell lines using the BD Clontech® Cancer Profiling Array II.

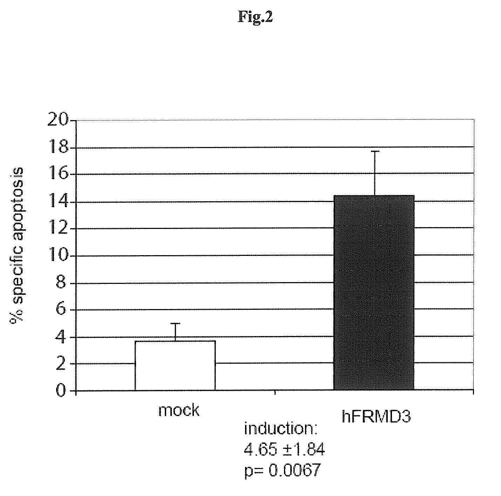

**Figure 2**: Apoptosis rate determined by Annexin V staining 24 hours after transfection of the cells with a negative control (mock) and hFRMD3. It is apparent that in the hFRMD3 transfected cells, a significantly increased rate of apoptosis is observed.

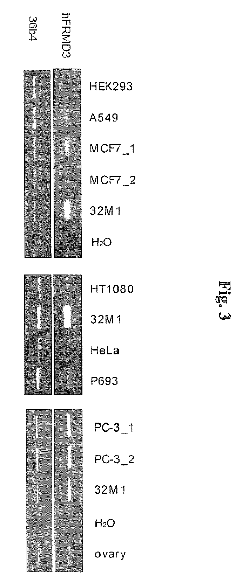

**Figure 3**: Expressionanalysis of various tumor cell lines. hFRMD3 expression has been observed in all cell lines except HEK293. It is of note that expression was even observed in PC-3 cell lines. A previous publication by Ni 1999, loc. cit., has reported no hFRMD3 expression in those cells. Normalization has been performed as described for Figure 1. HEK293 (embryonic kidney carcinoma cell line), A549 and P693 (pulmonary adenocarcinoma), MCF7 (breast carcinoma), HT1080 (pulmonary fibrosarcoma), 32M1 (pulmonary epithelial carcinoma), HeLa (cervix carcinoma), PC-3 (prostate carcinoma).

The invention will now be illustrated by the following Examples. The Examples shall, however, not be construed as to limit the scope of the invention in any respect.

### Example 1: Expressionanalysis of hFRMD3 in patient samples

Surgically resected NSCLC tissues had been obtained with informed consent of patients undergoing lobectomy at the Thoraxklinik Heidelberg according to the ethic votum 270/2001, version 2.0 (23.1.2005). A total of ten pSCC and two pAC as well as adjacent normal lung tissue of each patient was available for total RNA preparation. In addition one pSCC and one pSCLC and the respective adjacent normal tissue were fixed in paraformaldehyde and applied to cover slides for histochemical analysis.

Extracted RNAs from patients' tissues had been reverse transcribed into cDNA using oligo(dT) primers. RT-PCRs were performed with synthesized hFRMD3 primers (hFRMD3-ISH_3' sense tcc ccc agc gag caa gaa g SEQ ID NO: 3; and hFRMD3_ISH3' antisense acc cga ata tgg cca gtc aga atg SEQ ID NO: 4). As internal control acidic ribosomal phosphoprotein (h36b4 sense aac atg ctc aac atc tcc cc; SEQ ID NO: 5 and h36b4 antisense ccg act cct ccg act ctt c; SEQ ID NO: 6) specific primers were used. The PCR reactions were performed using 0.25 µl Taq polymerase, 50 pmol of sense and antisense primer each, 25 mM dNTP, 0.5 µl cDNA and 10% DMSO in 1x GB buffer (670 mM Tris pH 8.8, 166 mM (NH₄)₂SO₄, 67 mM MgCl2, 50 mM β-mercaptoethanol, 67 µM EDTA), according to the following protocol: denaturation at 90 °C for 30 seconds followed by an annealing step at 66 °C for hFRMD3 or 55 °C for h36b4 for 45 seconds and the elongation at 68 °C for 45 seconds. These steps were repeated 40 times for hFRMD3 and 33 times for h36b4. All amplicons, having a size of 520 bp for hFRMD3 and 400 bp for h36b4, were run on 2% agarose gels.
For S 100A9 the respective sense and antisense primers were used in the PCR reaction and the protocol described above was used with an annealing temperature of 58 °C and 33 cycles.

A comparison of the level of FRMD3 in normal and tumor tissue of each patient revealed a down regulation in seven of the seven tested pulmonary squamous cell carcinomas (pSCC) as well as in the two tested pulmonary adenocarcinomas (pAC) (Fig. 1A). In one pSCC tissue pair FRMD3 was neither detectable in the normal tissue nor in the tumor sample.

Moreover, the BD Clontech® Cancer Profiling Array II has been hybridized with a 1.2kb fragment of the coding region of the human FRMD3 gene. For colon carcinoma, a downregulation of FRMD3 expression was apparent in 7 out of 10 cases. For lung carcinoma, a down regulation was observed in 10 out of 10 cases (Fig. 1 B).

### Example 2: hFRMD3 expression results in apoptosis

In order to determine whether FRMD3 transfected HEK293 cells die from an increased apoptosis rate, Annexin V staining was performed 72h after transfection. Annexin V binds to negatively charged phosphatidylserines that are flipped from the inner to the outer part of the cell membrane in the early phase of apoptosis. Membrane-bound APC coupled Annexin V was detected by FACS analysis and the percentage of Annexin V positive cells determined.

For Annexin V staining cells were washed with PBS, resuspended in 100 µl Annexin buffer (10 mM HEPES, pH 7.4, 140 mM NaCl, 2.5 mM CaCl₂) and labeled with AnnexinV-APC (1:200, BD Biosciences Pharmingen) in the dark for 20 minutes at 4°C temperature. The staining mix was washed once with Annexin buffer before the percentage of Annexin V positive cells was determined by FACSCalibur at 660 nm.

As a positive control for apoptotic cells, HEK293 were irradiated with 40 J/m² UV light in the Stratalinker (Stratagene) and stained with Annexin V 24 hours after treatment. Compared to the 7% spontaneous apoptosis rate of HEK293, 30% of UV irradiates HEK293 cells were apoptotic.

72 hours post transfection, FRMD3 transfected cells exhibited an increased specific apoptosis (induction of apoptosis compared to spontaneous apoptosis rate of untransfected HEK293) of 14% compared to the 3% apoptotic cells in mock control (Fig. 2). This reveals an approximately 4.5fold induction of specific apoptosis upon forced FRMD3 expression (p-value = 0.00067).

### Example 3: Expressionanalysis of hFRMD3 in cell lines

Extracted RNAs from logarithmically growing cells had been reverse transcribed into cDNA using oligo(dT) primers. RNA from ovary was used as a positive control. RT-PCRs were performed with synthesized hFRMD3 primers (hFRMD3-ISH_3' sense tcc ccc agc gag caa gaa g SEQ ID NO: 3; and hFRMD3_ISH3' antisense acc cga ata tgg cca gtc aga atg SEQ ID NO: 4). As internal ribosomal protein P0 specific primers (36b4 sense aac atg ctc aac atc tcc cc; SEQ ID NO: 5 and 36b4 antisense ccg act cct ccg act ctt c; SEQ ID NO: 6) were used. The PCR reactions were performed using 0.25 µl Taq polymerase, 50 pmol of sense and antisense primer each, 25 mM dNTP, 0.5 µl cDNA and 10% DMSO in 1x GB buffer (670 mM Tris pH 8.8, 166 mM (NH₄)₂SO₄, 67 mM MgCl2, 50 mM β-mercaptoethanol, 67 µM EDTA), according to the following protocol: denaturation at 90 °C for 30 seconds followed by an annealing step at 66 °C for hFRMD3 or 55 °C for 36b4 for 45 seconds and the elongation at 68 °C for 45 seconds. These steps were repeated 40 times for hFRMD3 and 33 times for 36b4. All amplicons, having a size of 520 bp for hFRMD3 and 400 bp for 36b4, were run on 2% agarose gels.

## Claims

1. A pharmaceutical composition comprising
(i) a polynucleotide comprising
a) a nucleic acid having a sequence as shown in SEQ ID NO: 1,
b) a nucleic acid encoding a polynucleotide as shown in SEQ ID NO: 2,
c) a nucleic acid which is at least 60% identical to the nucleic acid of a) or b),
d) a nucleic acid which encodes a protein being at least 60% identical to the protein encoded by any one of a) to c), and
e) a nucleic acid encoding a biologically active fragment of any one of a) to d) or
(ii) a polypeptide encoded by the polynucleotides of (i) and, optionally, a pharmaceutically acceptable carrier.

2. Use of a polynucleotide or polypeptide as defined in claim 1 for the manufacture of a pharmaceutical composition for treating, preventing and/or ameliorating a hyperproliferative disorder.

3. The use of claim 2, wherein said hyperproliferative disorder is cancer.

4. The use of claim 3, wherein said cancer is a lung cancer or colon cancer.

5. A diagnostic composition comprising the polynucleotide or polypetide as defined in claim 1, an antibody which specifically binds to the polypeptide defined in claim 1 or an oligonucleotide specifically recognizing the polynucleotide as defined in claim 1.

6. A device for diagnosing a hyperproliferative disorder comprising the polynucleotide or polypeptide as defined in claim 1, an antibody which specifically recognizes the polypeptide as defined in claim 1 or an oligonucleotide specifically recognizing the polynucleotide as defined in claim 1.

7. Use of the polynucleotide or polypetide as defined in claim 1, an antibody which specifically recognizes the polypeptide defined in claim 1 or an oligonucleotide which specifically recognizes the polynucleotide of claim 1 for the preparartion of a diagnostic composition for diagnosing a hyperproliferative disorder.

8. A method for diagnosing a hyperproliferative disorder in a subject comprising the steps of:
a) determining the amount of the polynucleotide or polypeptide as defined in claim 1 in a sample of a subject; and
b) comparing said amount to a reference amount whereby the hyperproliferative disorder is diagnosed.

9. The method of claim 8, wherein said reference amount is obtained from a subject known to suffer from a hyperproliferative disorder.

10. The method of claim 9, wherein an amount essentially identical or less than the reference amount is indicative for the hyperproliferative disorder.

11. The method of claim 8, wherein the reference amount is obtained from a subject known not to suffer from a hyperproliferative disorder.

12. The method of claim 11, wherein an amount less than the reference amount is indicative for a hyperproliferative disorder.

13. The device of claim 6, the use of claim 7 or the method of any one of claim 8 to 12, wherein said hyperproliferative disorder is cancer.

14. The device, use or method of claim 13, wherein said cancer is a lung cancer or colon cancer.

15. A kit for carrying out the method of any one of claims 8 to 14 comprising the polynucleotide or polypetide as defined in claim 1, an antibody which specifically recognizes the polypeptide defined in claim 1 or an oligonucleotide which specifically recognizes the polynucleotide of claim 1 and instructions for carrying out the said method.


SEQUENCE LISTING

<110> Deutsches Krebsforschungszentrum, Stiftung des öffentlichen
Rechts

<120> Means and methods for diagnosing and treating cancer based on the
FRMD3 gene

<130> DK64116EP

<160> 6

<170> PatentIn version 3.3

<210> 1
<211> 1746
<212> DNA
<213> Homo sapiens

<400> 1
atgatccact ttcggagctc cagcgtcaaa tcgctcagcc aggagatgag atgcaccatc 60

cggctgctgg acgactcgga gatctcctgc cacatccaga gggaaaccaa agggcagttt 120

ctcattgacc acatctgcaa ctactacagc ctgctggaga aggactactt tggcattcgc 180

tatgtggacc cagagaagca aaggcactgg cttgaaccta acaagtccat cttcaagcaa 240

atgaaaactc atccaccata caccatgtgc tttagagtga aattctaccc acatgaaccc 300

ttgaagatta aagaagagct cacaagatac cttttatacc ttcagattaa aagggacatt 360

tttcatggcc acctgctgtg ctccttttct gatgctgcct acctgggtgc ctgtattgtt 420

caagctgagc ttggtgatta cgatcctaat gagcatcctg agaattacat cagtgagttt 480

gagattttcc ccaagcagtc acagaagctg gaaagaaaaa tagtggaaat tcataaaaat 540

gaactcaggg ggcagagccc accagttgct gaatttaact tgctcctgaa agctcacact 600

ttggaaacct acggggtgga tcctcaccca tgcaaggatt caacaggcac aacaacattt 660

ttaggattca cagctgcagg ctttgtggtc tttcagggaa ataagagaat ccatttgata 720

aaatggccag atgtctgcaa attgaagttt gaagggaaga cattttatgt gattggcacc 780

cagaaggaga aaaaagccat gttggcattc aatacttcaa caccagctgc ctgcaaacat 840

ctttggaagt gtggagtgga aaaccaggcc ttttataagt atgcaaaatc cagtcagatc 900

aagactgtat caagcagcaa gatatttttt aaaggaagta gatttcgata tagtgggaaa 960

gttgccaaag aggtggtgga ggccagttcc aagatccaga gggagcctcc tgaggtgcac 1020

agagccaaca ttactcagag ccgcagttcc cactccttga acaaacagct catcattaac 1080

atggaacccc tgcagcccct gcttccttcc cccagcgagc aagaagaaga acttcctctg 1140

ggtgagggtg ttccattgcc taaagaggag aacatttctg ctcccttgat ctccagctcc 1200

ccagtgaagg cagcccggga gtatgaagat ccccctagtg aagaggaaga taaaataaaa 1260

gaagaacctt taaccatctc tgaactagtg tacaacccaa gtgccagcct gctccccacc 1320

cctgtggatg acgatgagat tgacatgctc tttgactgtc cttctaggct tgagttggaa 1380

agagaagaca cagattcatt tgaggatctg gaagcagatg aaaacgcctt tttgattgct 1440

gaagaagagg agctgaagga ggctcgccgt gctttgtcgt ggagctacga cattctgact 1500

ggccatattc gggtgaaccc actggtcaag agtttttcca ggctccttgt ggtgggcctg 1560

ggactgctgc tctttgtatt tcccctgctc ctcctccttt tggagtcagg tattgatctc 1620

tccttcttat gcgaaatccg ccagacacca gagtttgagc agtttcacta tgaatactac 1680

tgtcccctca aggagtgggt ggctgggaaa gtccacctca tcctctacat gctgggttgc 1740

tcatga 1746

<210> 2
<211> 581
<212> PRT
<213> Homo sapiens

<400> 2

Met Ile His Phe Arg Ser Ser Ser Val Lys Ser Leu Ser Gln Glu Met
1               5                   10                  15

Arg Cys Thr Ile Arg Leu Leu Asp Asp Ser Glu Ile Ser Cys His Ile
            20                  25                  30

Gln Arg Glu Thr Lys Gly Gln Phe Leu Ile Asp His Ile Cys Asn Tyr
        35                  40                  45

Tyr Ser Leu Leu Glu Lys Asp Tyr Phe Gly Ile Arg Tyr Val Asp Pro
    50                  55                  60

Glu Lys Gln Arg His Trp Leu Glu Pro Asn Lys Ser Ile Phe Lys Gln
65                  70                  75                  80

Met Lys Thr His Pro Pro Tyr Thr Met Cys Phe Arg Val Lys Phe Tyr
                85                  90                  95

Pro His Glu Pro Leu Lys Ile Lys Glu Glu Leu Thr Arg Tyr Leu Leu
            100                 105                 110

Tyr Leu Gln Ile Lys Arg Asp Ile Phe His Gly His Leu Leu Cys Ser
        115                 120                 125

Phe Ser Asp Ala Ala Tyr Leu Gly Ala Cys Ile Val Gln Ala Glu Leu
    130                 135                 140

Gly Asp Tyr Asp Pro Asn Glu His Pro Glu Asn Tyr Ile Ser Glu Phe
145                 150                 155                 160

Glu Ile Phe Pro Lys Gln Ser Gln Lys Leu Glu Arg Lys Ile Val Glu
                165                 170                 175

Ile His Lys Asn Glu Leu Arg Gly Gln Ser Pro Pro Val Ala Glu Phe
            180                 185                 190

Asn Leu Leu Leu Lys Ala His Thr Leu Glu Thr Tyr Gly Val Asp Pro
        195                 200                 205

His Pro Cys Lys Asp Ser Thr Gly Thr Thr Thr Phe Leu Gly Phe Thr
    210                 215                 220

Ala Ala Gly Phe Val Val Phe Gln Gly Asn Lys Arg Ile His Leu Ile
225                 230                 235                 240

Lys Trp Pro Asp Val Cys Lys Leu Lys Phe Glu Gly Lys Thr Phe Tyr
                245                 250                 255

Val Ile Gly Thr Gln Lys Glu Lys Lys Ala Met Leu Ala Phe Asn Thr
            260                 265                 270

Ser Thr Pro Ala Ala Cys Lys His Leu Trp Lys Cys Gly Val Glu Asn
        275                 280                 285

Gln Ala Phe Tyr Lys Tyr Ala Lys Ser Ser Gln Ile Lys Thr Val Ser
    290                 295                 300

Ser Ser Lys Ile Phe Phe Lys Gly Ser Arg Phe Arg Tyr Ser Gly Lys
305                 310                 315                 320

Val Ala Lys Glu Val Val Glu Ala Ser Ser Lys Ile Gln Arg Glu Pro
                325                 330                 335

Pro Glu Val His Arg Ala Asn Ile Thr Gln Ser Arg Ser Ser His Ser
            340                 345                 350

Leu Asn Lys Gln Leu Ile Ile Asn Met Glu Pro Leu Gln Pro Leu Leu
        355                 360                 365

Pro Ser Pro Ser Glu Gln Glu Glu Glu Leu Pro Leu Gly Glu Gly Val
    370                 375                 380

Pro Leu Pro Lys Glu Glu Asn Ile Ser Ala Pro Leu Ile Ser Ser Ser
385                 390                 395                 400

Pro Val Lys Ala Ala Arg Glu Tyr Glu Asp Pro Pro Ser Glu Glu Glu
                405                 410                 415

Asp Lys Ile Lys Glu Glu Pro Leu Thr Ile Ser Glu Leu Val Tyr Asn
            420                 425                 430

Pro Ser Ala Ser Leu Leu Pro Thr Pro Val Asp Asp Asp Glu Ile Asp
        435                 440                 445

Met Leu Phe Asp Cys Pro Ser Arg Leu Glu Leu Glu Arg Glu Asp Thr
    450                 455                 460

Asp Ser Phe Glu Asp Leu Glu Ala Asp Glu Asn Ala Phe Leu Ile Ala
465                 470                 475                 480

Glu Glu Glu Glu Leu Lys Glu Ala Arg Arg Ala Leu Ser Trp Ser Tyr
                485                 490                 495

Asp Ile Leu Thr Gly His Ile Arg Val Asn Pro Leu Val Lys Ser Phe
            500                 505                 510

Ser Arg Leu Leu Val Val Gly Leu Gly Leu Leu Leu Phe Val Phe Pro
        515                 520                 525

Leu Leu Leu Leu Leu Leu Glu Ser Gly Ile Asp Leu Ser Phe Leu Cys
    530                 535                 540

Glu Ile Arg Gln Thr Pro Glu Phe Glu Gln Phe His Tyr Glu Tyr Tyr
545                 550                 555                 560

Cys Pro Leu Lys Glu Trp Val Ala Gly Lys Val His Leu Ile Leu Tyr
                565                 570                 575

Met Leu Gly Cys Ser
            580

<210>  3
<211>  19
<212>  DNA
<213>  artificial sequence

<220>
<223>  primer hFRMD3-ISH_3' sense

<400>  3
tcccccagcg agcaagaag                                                    19

<210>  4
<211>  24
<212>  DNA
<213>  artificial sequence

<220>
<223>  primer hFRMD3_ISH3` antisense

<400>  4
acccgaatat ggccagtcag aatg                                              24

<210>  5
<211>  20
<212>  DNA
<213>  artificial sequence

<220>
<223>  primer 36b4 sense

<400>  5
aacatgctca acatctcccc                                                   20

<210>  6
<211>  19
<212>  DNA
<213>  artificial sequence

<220>
<223>  primer 36b4 antisense

<400>  6
ccgactcctc cgactcttc                                                    19